(19) Europäisches Patentamt
European Patent Office
Office européen des brevets

(11) Veröffentlichungsnummer: **0 073 331**
**B1**

(12) **EUROPÄISCHE PATENTSCHRIFT**

(45) Veröffentlichungstag der Patentschrift: **09.04.86**

(21) Anmeldenummer: **82106482.1**

(22) Anmeldetag: **19.07.82**

(51) Int. Cl.⁴: **C 07 D 249/08**, C 07 D 233/60, A 01 N 43/64, A 01 N 43/50 // C07C49/255, C07C49/227, C07C43/225, C07C11/12

(54) **5-Aryloxy-5-azolyl-3,3-dimethyl-1-penten-4-one und -ole, Verfahren zu ihrer Herstellung sowie ihre Verwendung als Fungizide.**

(30) Priorität: **01.08.81 DE 3130435**

(43) Veröffentlichungstag der Anmeldung: **09.03.83 Patentblatt 83/10**

(45) Bekanntmachung des Hinweises auf die Patenterteilung: **09.04.86 Patentblatt 86/15**

(84) Benannte Vertragsstaaten: **AT BE CH DE FR GB IT LI NL**

(56) Entgegenhaltungen:
**EP-A-0 001 414**
**EP-A-0 042 980**
**EP-A-0 052 424**
**DE-A-2 632 602**
**DE-A-2 632 603**
**DE-A-2 918 801**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Jäger, Gerhard, Dr., Gellertstrasse 18, D-5090 Leverkusen (DE)**
Erfinder: **Jautelat, Manfred, Dr., Muellersbaum 28, D-5093 Burscheid (DE)**
Erfinder: **Büchel, Karl Heinz, Prof.Dr., Dabringhausener Strasse 42, D-5093 Burscheid (DE)**
Erfinder: **Brandes, Wilhelm, Dr., Eichendorffstrasse 3, D-5653 Leichlingen (DE)**
Erfinder: **Frohberger, Paul-Ernst, Dr., Willi-Baumeister-Strasse 5, D-5090 Leverkusen (DE)**

**EP 0 073 331 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue 5-Aryloxy-5-azolyl-3,3-di-methyl-1-penten-4-one und -ole, bin Verfahren zu ihrer Herstellung sowie ihre Verwendung als Fungizide.

Es ist bereits bekannt geworden, daß im Phenylteil substituierte 4-Chlor(Brom)-3,5-dimethyl-1-imidazolyl-(1,2,4-triazolyl)-1-phen-oxy-butan-2-one und -ole im allgemeinen gute fungizide Eigenschaften aufweisen (vergleiche DE-A- 2 632 603 und DE-A-2 632 602. Die Wirkung ist jedoch, insbesondere bei niedrigen Aufwendmengen und -konzentrationen, in einigen Anwendungsbereichen nicht immer voll befriedigend, obwohl die letztgennanten Verbindungen den ebenfalls bekannt gewordenen Triazolyl-O,Nacetalen (vgl, hierzu EP-A 0 001 414) nach überlegen sind, Schließ-lich sind nach fungizid wirksame 1,2,4-Triazoyl-carbinolether beschrieben (vgl. DE-A- 2 918 801), die jedoch in einzelnen Anwendungsbereichen nicht immer voll zufriedenstellend wirksam sind.

Es wurden neue 5-Aryloxy-5-azolyl-3,3-dimethyl-1-penten-4-one und -ole der Formel (I)

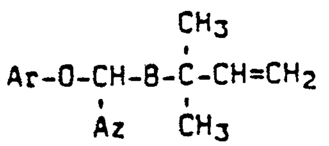

(I)

in welcher

Ar für gegebenenfalls einfach oder mehrfach, gleich oder Verschieden substituiertes Phenyl steht, wobei als Substiuenten Halogen, Alkyl mit 1 bis 4 Kohlenstoffatomen, Halogenalkyl mit 1 bis 2 Kohlenstoff- und 1 bis 5 gleichen oder Verechiedenen Halogenatomen, Alkoxycarbonyl mit 1 bis 4 Kohlenstoffatomen im Alkylteil, Nitro, Cyano oder gegebenenfalls durch Halogen und/oder Alkyl mit 1 bis 2 Kohlenstoffatomen substituiertes Phenyl genannt seien,

Az für 1,2,4-Triazol-1-yl, 1,2,4-Triazol-4-yl oder Imidazol-1-yl steht und

B für die Ketogruppe oder eine CM(OH)-Gruppierung steht,

sowie deren physiologisch verträglichen Säureadditionssalze und Metallsalz-Komplexe gefunden.

Diejenigen Verbindungen der Formel (I), in welchen B für die CH(OH)-Gruppe steht, besitzen zwei asymmetrische Kohlenstoffatome; sie können deshalb in den beiden geometrischen Isomeren (erythro- und threo-Form) vorliegen, die in unterechiedlichen Mengenverhältnissen anfallen können. In beiden Fällen liegen sie als optische Isomeren vor. Sämtliche Isomeren werden erfindungegemäß beansprucht.

Weiterhin wurde gefunden, daß man die 5-Aryloxy-5-azolyl-5,3-di-methyl-1-penten-4-one und -ole der Formel (I) erhält, wenn man Halogenetherketone der Formel (II)

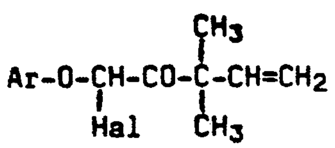

(II)

in welcher

Ar die oben angegebene Bedeutung hat und

Hal für Chlor oder Brom steht,

mit 1,2,4-Triazol oder Imidazol in Gegenwart eines Säurebindemittels und gegebenenfalls in Gegenwart eines Verdünnungsmittels umsetzt und gegebenenfalls noch die erhaltenen Keto-Derivate der Formel (I) nach bekannten Methoden in üblicher Weise reduziert.

An die so erhaltenen Verbindungen der Formel (I) kann gegebenenfalls anschließend eine Säure oder ein Metallsalz addiert werden.

Die neuen 5-Aryloxy-5-azolyl-5,3-dimethyl-1-penten-4-one und -ole der Formel (1) weisen starke fungizide Eigenschaften auf. Dabei zeigen überraschenderweise die erfindungsgemäßen Verbindungen eine bessere fungizide Wirksamkeit als die aus dem Stand der Technik bekannten im Phenylteil substituierten 4-Chlor(Brom)-5,3-dimethyl-1-imida-zolyl(1,2,4-triazolyl)-1-phenoxy-butan-2-one und -ole, welche chemiech und wirkungemäßig naheliegende Verbindungen sind. Die erfindungsge-mäßen Stoffe stellen somit eine Bereicherung der Technik dar.

Die erfindungsgemäßen 5-Aryloxy-5-azolyl-3,3-dimethyl-1-pentan-4-one und -ole sind durch die Formel (1) allgemein definiert.

Bevorzugt sind diejenigen Verbindungen der Formel (I), in denen

Ar für gegebenenfalls einfach bis dreifach, gleich oder verschieden substituiertes Phenyl steht, wobei als Substituenten genannt seien: Fluor, Chlor, Brom, Methyl, Ethyl, Trifluormethyl, Methoxycarbonyl, Ethoxycarbonyl, Nitro, Cyano, Phenyl und Chlorphenyl sowie

Az und B für die in der Erfindungsdefinition angegebenen Bedeutungen stehen.

Im einzelnen seien außer den bei den Herstellungsbeispielen genannten Verbindungen die folgenden Verbindungen der allgemeinen Formel (I) genannt:

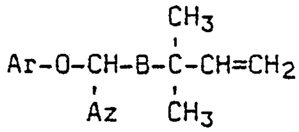

(I)

| Ar | Az | B |
|---|---|---|
| 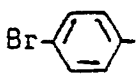 | 1,2,4-Triazol-1-yl | CO und CH(OH) |
| 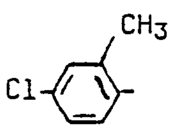 | 1,2,4-Triazol-1-yl | CO und CH(OH) |
| 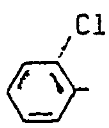 | 1,2,4-Triazol-1-yl | CO und CH(OH) |
| 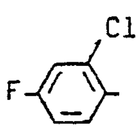 | 1,2,4-Triazol-1-yl | CO-und CH(OH) |
| 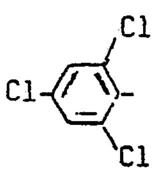 | 1,2,4-Triazol-1-yl | CO und CH(OH) |

| Ar | Az | B |
|---|---|---|
| 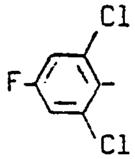 | 1,2,4-Triazol-1-yl | CO und CH(OH) |
| 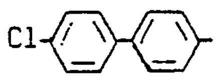 | 1,2,4,-Triazol-1-yl | CO und CH(OH) |
| 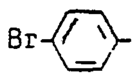 | Imidazol-1-yl | CO und CH(OH) |
| 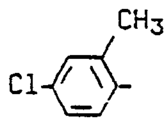 | Imidazol-1-yl | CO und CH(OH) |
| 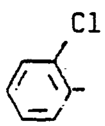 | Imidazol-1-yl | CO und CH(OH) |
| 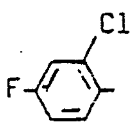 | Imidazol-1-yl | CO und CH(OH) |
| 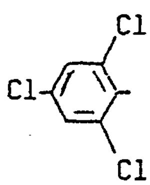 | Imidazol-1-yl | CO und CH(OH) |
| 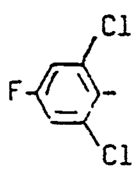 | Imidazol-1-yl | CO und CH(OH) |
| 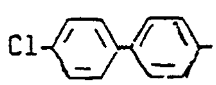 | Imdiazol-1-yl | CO und CH(OH) |

Vervendet man beispielsweise 5-Brom-5-(4-chlorphenoxy)-3,3-dimethyl-1-penten-4-on und Imidazol als Ausgangsstoffe, so kann der Reaktionsablauf durch das folgende Formelschema wiedergegeben werden:

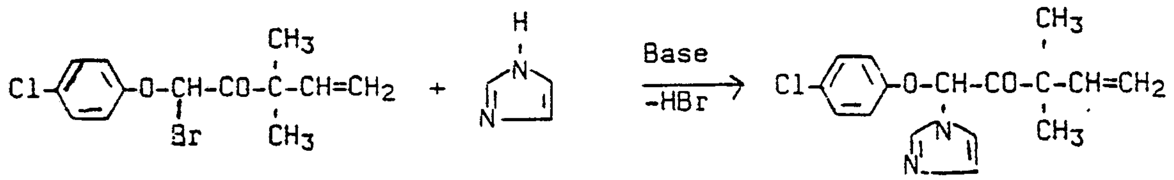


Verwendet man beispielsweise 5-(4-Chlorphenoxy)-3,5-dimethyl-5-(imi-dazol-1-yl)-1-penten-4-on und Natriumborhydrid als Ausgangsstoffe, so kann der Reaktionsablauf der Reduktion durch das folgende Formelschema wiedergegeben werden:

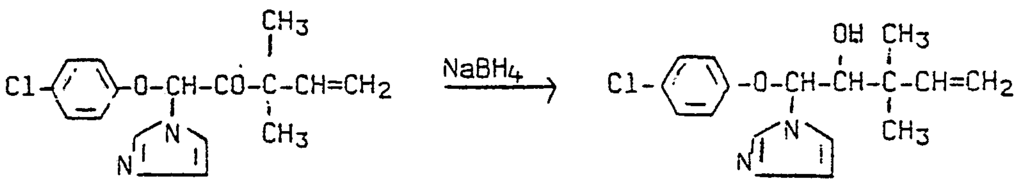


Die bei der Durchführung des erfindungsgemäßen Verfahrens als Ausgangsstoffe zu verwendenden Halogenether-Ketone sind durch die Formel (II) allgemein definiert. In dieser Formel steht Ar vorzugsweise für die Reste, die bereits im Zusammenhang mit der Beschreibung der erfindungsgemäßen Stoffe der Formel (I) vorzugsweise für diesen Substituenten genannt wurden.

Die Halogenetherketone der Formel (II) sind noch nicht bekannt; sie können jedoch nach bekannten Verfahren hergestellt werden, indem man z.B. bei Etherketonen der Formel (III)

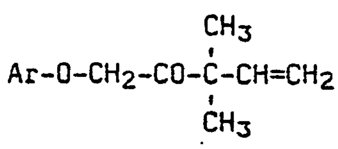

(III)

in welcher

Ar die oben angegebene Bedeutung hat,

eines der beiden aktiven Wasserstoffatome in üblicher Weise gegen Chlor oder Brom austauscht. Die entstehenden Halogenetherketone der Formel (II) können ohne Isolierung direkt weiter umgesetzt werden (vergleiche auch die Herstellungsbeispiele).

Die Etherketone der Formel (III) sind ebenfalls noch nicht bekannt; sie sind jedoch teilweise Gegenstand einer eigenen Patentanmeldung (DE-A 3 101 143 vom 16.1.1981 Die Etherketone der Formel (III) können nach dem dort angegebenen Eintopf-Verfahren erhalten werden, indem man Aryloxy-dien-Derivate der Formel (IV)

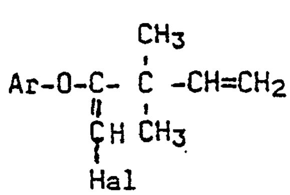

(IV)

in welcher

Ar und Hal die oben angegebene Bedeutung haben,

im Temperaturbereich zwischen +20 und 150° C einer sauren Hydrolyse unterwirft und anschließend nach Hinzufügen basischer Stoffe, wie z.B. Kaliumcarbonat, bei schwach alkalischer Reaktion die Umsetzung im genannten Temperaturbereich zu Ende führt.

Die Aryloxy-dien-Derivate der Formel (IV) können in einfacher Weise aus Olefinen durch Halogenwasserstoff-Addition, nachfolgende Addition an Vinylidenhalogenid in Gegenwart von sauren Katalysatoren (z.B. Aluminiumchlorid) und Umsetzung des erhaltenen Adduktes mit Alkaliphenolaten erhalten werden (DE-A- 3 029 270 vom 1.8.1980 und DE-A- 5 049 461 vom 50.12.1980

Die Etherketone der Formel (III) können auch erhalten werden, indem man entsprechende Phenole mit 5-Chlor(Brom)-3,3-dimethyl-1-penten-4-on der Formel V

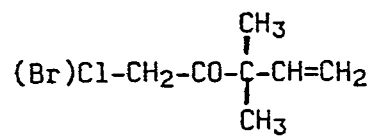

(V)

in Gegenwart einer starken Base, wie z.B. Kaliumcarbonat, und in Gegenwart eines inerten organischen Lösungsmittels, wie z.B. Aceton, bei Temperaturen zwischen 20 und 100° C umsetzt.

Das 5-Chlor(Brom)-3,5-dimethyl-1-penten-4-on der Formel (V) ist noch nicht bekannt; es ist jedoch Gegenstand einer eigenen älteren Patentammeldung

DE-A- 3 049 461 vom 30.12.1980 und wird erhalten, indem man z.B. 1,1-Dichlor-3,3-dimethyl-1,4-pentadien mit Alkaliphenolaten umsetzt und anschließend das dabei erhaltene Reaktionsprodukt einer sauren Hydrolyse unterwirft (vergleiche auch die Herstellungsbeispiele).

Für die erfindungsgemäße Umsetzung kommen als Verdünnungsmittel inerte organiache Lösungsmittel infrage. Hierzu gehören vorzugaweiae Ketone, wie Diethylketon und insbesondere Aceton und Methylethylketon; Nitrile,wie Propionitril, insbesondere Acetonitril; Alkohole, wie Ethanol oder Isopropanol; Ether, wie Tetrahydrofuran oder Dioxan; Benzol,Toluol, Formamide, wie insbesondere Dimethylformamid; und halogenierte Kohlenwasserstoffe.

Die erfindungagemäße Umsetzung wird in Gegenwart eines Säurebindera vorgenommen. Man kann alle üblicherweise verwendbaren anorganiachen oder organiachen Säurebinder zugeben, wie Alkalicarbonate, beiapielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan. Vorzugsweise verwendet man einen entaprechenden Ueberachuß an Triazol bzw. Imidazol.

Die Reaktionstemperaturen können in einem größeren Bereich variiert werden. Im allgemeinen arbeitet man zwischen etwa 20 bis etwa 150° C, vorzugsweise bei 60 bis 120° C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens setzt man auf 1 Mol der Verbindungen der Formel (II) vorzugsweise 2 Mol Triazol bzw. Imidazol und 1 bis 2 Mol Säurebinder ein. Zur Isolierung der Verbindungen der Formel (I) wird das Lösungsmittel abdestilliert, der Rückstand mit einem organischen Solvens aufgenommen und mit Wasser gewaschen. Die organische Phase wird über Natriumsulfat getrocknet und im Vakuum vom Lösungsmittel befreit. Der Rückstand wird durch Destillation bzw. Umkristallisation oder Salzbildung und Umkristallisation gereinigt.

Die erfindungsgemäße Reduktion erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei 0 bis 30° C, vorzugsweise bei 0 bis 20° C durchgeführt. Hierzu setzt man auf 1 Mol des Ketons der Formel (I) etwa 1 Mol eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120° C, vorzugsweise bei 50 bis 100° C. Zur Durchführung der Reaktion setzt man auf 1 Mol des Ketons der Formel (I) etwa 0,5 bis 2 Mol Aluminiumisopropylat ein. Zur Isolierung der reduzierten Verbindungen der Formel (I) wird das überschüssige Lösungsmittel im Vakuum entfernt und die entstandenen Aluminium-Verbindungen mit verdünnter Schwefelsäure oder Natronlauge zersetzt. Die weitere Aufarbeitung erfolgt in üblicher Weise.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel oder durch Umkristallisation gereinigt werden.

Zur Herstellung von Metallsalz-Komplexen der Verbindungen der Formel (I) kommen vorzugsweise Salze von Metallen der II. bis IV.-Hauptund der I. und II. sowie IV. bis VIII. Nebengruppe infrage, wobei Kupfer, Zink, Mangan, Magnesium, Zinn Eisen und Nickel beispielhaft genannt seien Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalzkomplexe in bekannter Weise, z.B. durch Abfiltrieren, isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilzeeingesetzt werden, die echte Mehltauerkrankungen hervorrufen, so zur Bekämpfung von Erysiphe-Arten, wie z.B. gegen den Erreger des Gerstenmehltaus (Erysiphe graminis), von Sphaerotheca-Arten, wie z.B. gegen den Erreger des Gurkenmehltaus (Sphaerotheca fuliginea), sowie von Podosphaera-Arten, wie z.B. gegen den Erreger des Apfelmehltaus (Podosphaera Leucotricha); außerdem auch zur Bekämpfung von Reiskrankheiten, wie z.B. Pyricularia oryzae. Daneben besitzen die er-findungsgemäßen Stoffe breite fungizide invitro-Wirkung.

In entsprechenden Aufwandmengen zeigen die erfindungsgemäßen Stoffe auch herbizide bzw. pflanzenwachstumsregulierende Wirksamkeit.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmittein, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittei können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthalinc, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnuß-schalen, Maiskolben und Tabakstengel; als Emulgierund/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche,in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflenzenteilen können die Wirkstoffkonzentrationen in den Anwendungsformen in einem größeren Bersich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

**Herstellungsbeispiele**

**Beispiel 1**

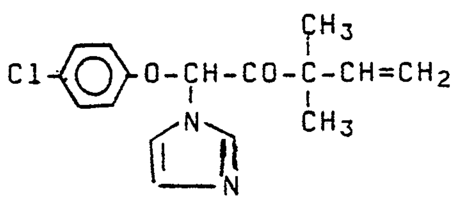

Zu einer Lösung von 28,6g (0,42 Mol) Imidazol in 250 ml Acetonitril werden innerhalb 5 Minuten 23 g rohes 5-Brom-5-(4-chlorphenoxy)-3,3-dimethyl-1-penten-4-on, gelöst in 50 ml Acetonitril, getropft. Man erhitzt anschliessend noch 3 Stunden zum Sieden und engt die Lösung unter vermindertem Druck ein. Der ölige Rückstand wird in 200 ml Essigester aufgenommen und dreimal mit je 50 ml Wasser gewaschen. Die organische Phase wird nach dem Trocknen über wasserfreiem Natriumsulfat eingedampft, der Rückstand in 200 ml Aceton aufgenommen und mit einer filtrierten Lösung von 20,2g (0,07 Mol) Naphthalindi-sulfonsäure-(1,5) in 100 ml Aceton versetzt. Das ausgeschiedene Salz wird abgenutscht, mit 100 ml Wasser und 100 ml Dichlormethan versetzt und anschließend mit 10 %-iger Sodalösung alkalisch gestellt. Die organische Phase wird abgetrennt und eingeengt. Der ölige Rückstand kristallisiert beim Anreiben mit Ether/Petrolether. Man erhält 7,4g (34,7 % der Theorie) 5-(4-Chlorphenoxy)-3,5-dimethyl-5-(imidazol-1-yl)-1-penten-4-on vom Schmelzpunkt 72-74° C.

**Herstellung des Ausgangsproduktes**

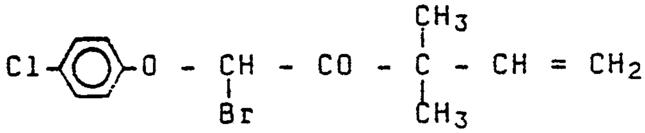

Zu einer Lösung von 16,7g (0,07 Mol) S-(4-Chlorphenoxy)-3,3-dimethyl-1-penten-4-on und einer Spatelspitze Azoisobutyronitril in 200 ml Tetrachlormethan werden 12,5g (0,07 Mol) N-Bromsuccinimid gegeben. Das Gemisch

wird anschliessend B Stunden unter UV-Belichtung zum Sieden erhitzt. Man kühlt dann auf 5°C ab und filtriert vom ausgeschiedenen Succinimid ab. Das Filtrat wird im Vakuum eingeengt. Man erhält 23 g rohes 5-Brom-5-(4-chlorphenoxy)-3,5-dimethyl-1-penten-4-on als Oel, das direkt weiter umgesetzt wird.

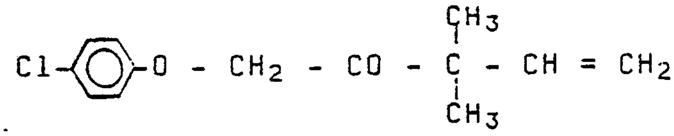

92,9g (0,725 Mol) 4-Chlorphenol werden mit 99,7g (0,725 Mol) fein gepulvertem Kaliumcarbonat in 500 ml Aceton zum Sieden erhitzt. Dazu werden innerhalb von 50 Minuten 106g (0,723 Mol) 5-Chlor-3,5-dimethyl-1-penten-4-on unter kräftigem Rühren getropft. Nach vierstündigem Erhitzen unter Rückfluß kühlt man das Gemisch auf 20°C ab, filtriert von den anorganischen Salzen ab und engt das Filtrat ein. Nach Destillation des flüssigen Rückstandes erhält man 136,3 g (79 % der Theorie) 5-(4-Chlor-phenoxy)-5,5-dimethyl-1-penten-4-on vom Siedepunkt 111-115°C/0,07 Torr.

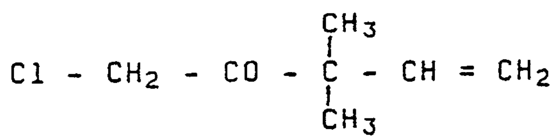

57g (0,256 Mol) 1-Chlor-3,3-dimethyl-2-phenoxy-1,4 pentadien werden im Gemisch aus 250 ml Ameisensäure und 50 ml konzentrierte Salzsäure während 1 Stunde auf 40°C erwärmt. Dann wird mit 400 ml Methylenchlorid und Eis verdünnt und dreimal mit 2n Natronlauge ausgeschüttelt. Nach dem Trocknen der Methylenchloridphase über Natriumsulfat wird das Lösungsmittel entfernt. Es bleiben 36 g Produkt (das sind 96 % der Theorie) zurück, welches destilliert wird. Man erhält 32,2g (86 % der Theorie) 5-Chlor-3,3-dimethyl-1-penten-4-on vom Siedepunkt 81-84°C/24 Torr.

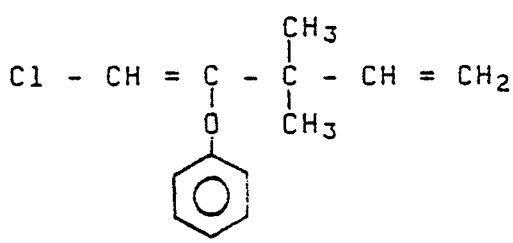

116g (1 Mol) Natrium-phenolat und 82,5g (0,5 Mol) 1,1-Dichlor-5,5-dimethyl-1,4-pentadien werden in 500 ml Dimethylformamid 8 Stunden unter Rückfluß erhitzt. Die Lösung wird mit Methylenchlorid verdünnt und mit verdünnter Natronlauge ausgeschüttelt. Nach Trocknen der Methylenchloridphase über Natriumsulfat wird das Lösungsmittel im Vakuum abgezogen. Das zurückbleibende Rohprodukt wird im Vakuum destilliert. Man erhält 95,6g (84 % der Theorie) 1-Chlor-3,3-dimethyl-2-phenoxy-1,4-pentadien vom Siedepunkt 80-90°C/0,5 Torr.

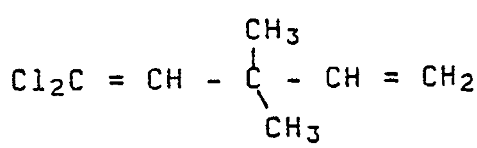

Zu 1000ml Chinolin werden bei 225 bis 230°C langsam 201,5 g (1 Mol) 3,3-Dimethyl-1,1,5-trichlor-1-penten eingetropft und über den Kopf einer Kolonne wird gleichzeitig Destillat abgenommen. Man steigert die Temperatur bis zum Sieden des Chinolins und isoliert insgesamt 126g Destillat, das nochmals fraktioniert wird. Man erhält 121g (75 % der Theorie) 1,1-Dichlor-3,5-dimethyl-1,4-pentadien vom Siedepunkt 49-55°C/20 Torr.

**Beispiel 2 und 3**

Bsp.2 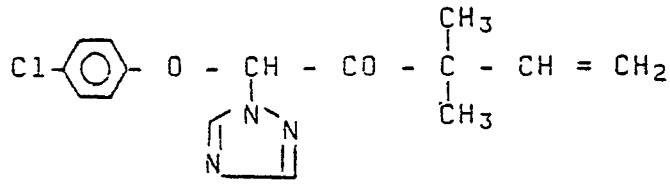

Bsp.3 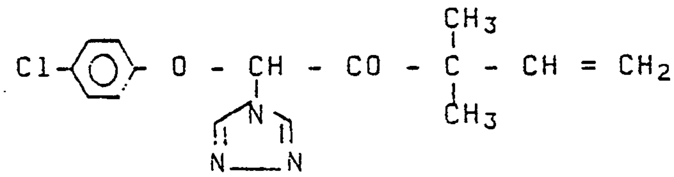

Zu einer Lösung von 115,9g (1,68 Mol) 1,2,4-Triazol in 1000 ml Acetonitril werden bei 50°C 89 g (0,28 Mol) 5-Brom-5-(4-chlorphenoxy)-3,5-dimethyl-1-penten-4-on, gelöst in 120 ml Acetonitril, innerhalb von 50 Minuten getropft. Nach dreistündigem Erhitzen der Lösung zum Sieden wird das Lösungsmittel abdestilliert und der Rückstand in 500 ml Essigester aufgenommen. Es wird dreimal mit je 50 ml Wasser gewaschen und die organische Phase nach dem Trocknen über wasserfreiem Natriumsulfat unter gemindertem Druck eingedampft. Das verbleibende Oel wird in 400 ml Aceton gelöst und mit einer Lösung von 80,6g Naphthalindisulfonsäure-(1,5) in 400 ml Aceton versetzt. Das auskristallisierte Salz wird abgenutscht, mit Aceton gewaschen, mit je 250 ml Wasser und Dichlormethan verrührt und anschließend mit einer 10 %-igen Natriumcarbonatlösung alkalisch gestellt. Das nach dem Eindampfen der organsichen Phase zurückbleibende Oel wird in wenig Ether gelöst und anschließend langsam mit Petrolether versetzt. Das dabei auskristallisierende Produkt wird abfiltriert und mit wenig Ether/Petrolether gewaschen. Man erhält 17,1g (20 % der Theorie) 5-(4-Chlorphenoxy)-3,3-dimethyl-5-(1,2,4-triazol-4-yl)-1-penten-4-on (Beispiel 3) vom Schmelzpunkt 124-126°C.

Das Filtrat wird eingeengt und der ölige Rückstand in Chloroform/Essigester (1:2) aufgenommen. Nach Filtrieren über eine Kieselgelsäule erhält man 17,5g (20,4 % der Theorie) 5-(4-Chlorphenoxy)-3,3-dimethyl-5-(1,2,4-triazol-1-yl)-1-penten-4-on (Beispiel 2) vom Brechungsindex $n^{20}_{D}$-1,5419.

**Beispiel 4**

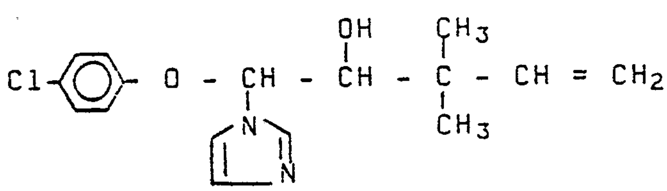

Zu einer Lösung von 15,5g (0,05 Mol) 5-(4-Chlorphenoxy)-5,5-dimethyl-5-(imidazol-1-yl)-1-penten-4-on (Beispiel 1) in 150 ml Methanol werden unter Außenkühlung portionsweise 5,8g (0,1 Mol) Natriumboranat eingetragen. Nach 6 Stunden wird mit verdünnter Essigsäure auf pH 6 eingestellt und das Reaktionsgemisch unter vermindertem Druck eingedampft. Der Rückstand wird in Essigester aufgenommen, die Lösung einmal mit verdünnter Natriumhydrogencarbonatlösung und dreimal mit Wasser gewaschen, über Natriumsulfat getrocknet und danach im Vakuum eingeengt. Der ölige Rückstand wird in wenig Ether gelöst und mit Petrolether versetzt. Der entstehende Niederschlag wird abgetrennt, mit wenig Petrolether gewaschen und getrocknet. Man erhält 12,2g (797% der Theorie) 5-(4-Chlorphenoxy)-3,3-dimethyl-5-(imidazol-1-yl)-1-penten-4-ol vom Schmelzpunkt 88-90°C.

In entsprechender Weise werden die Verbindungen der folgenden Tabelle 1 der allgemeinen Formel

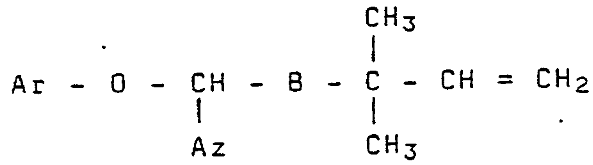 (I)

erhalten:

| Bsp. Nr. | Ar | Az | B | physikalische Konstante |
|---|---|---|---|---|
| 5 | 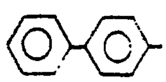 | 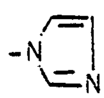 | CO | Harz |
| 6 | 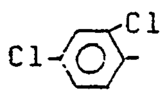 | 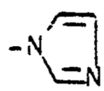 | CO | Harz |
| 7 | 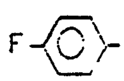 | 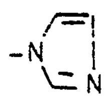 | CO | $n_D^{20}$ =1,5301 |
| 8 | 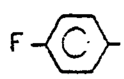 | 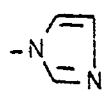 | CO | Fp:242°C(Zers.) (x 1/2 NDS)* |
| 9 | 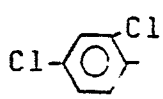 | 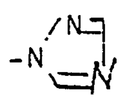 | CO | Oel |
| 10 | 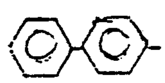 | 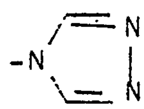 | CO | Fp:152-53°C |

| Bsp. Nr. | Ar | Az | B | physikal. Konstante |
|---|---|---|---|---|
| 11 | 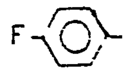 | 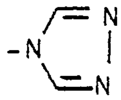 | CO | Fp:91-94°C |
| 12 | 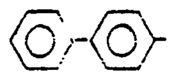 | 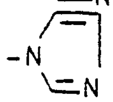 | CH(OH) | Fp: 132°C |
| 13 | 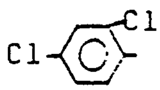 | 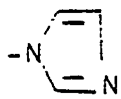 | CH(OH) | Oel |
| 14 | 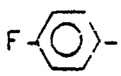 | 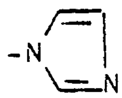 | CH(OH) | Oel |
| 15 | 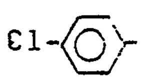 | 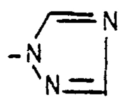 | CH(OH) | Harz |
| 16 | 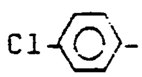 | 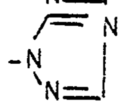 | CH(OH) | Fp:167-69°C (x1/2 NDS)* |
| 17 | 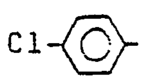 | 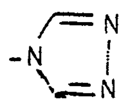 | CH(OH) | Fp:200-01°C/ (Form A)** |
| 18 | 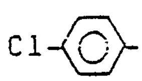 | 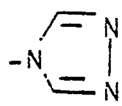 | CH(OH) | Fp:170-90°C |

* NDS = Naphthalindisulfonsäure-(1,5)
**FormA= Eine der beiden möglichen geometrischen Isomeren

**Verwendungsbeispiele**

In den nachfolgenden Beispielen werden die nachstehend angegebenen Verbindungen als Vergleichssubstanzen eingesetzt:

(A) 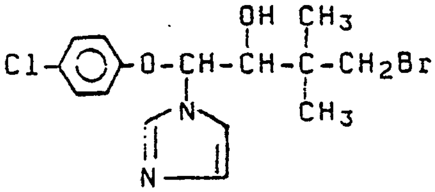 gemäß DE-A-2632602 (Beispiel 3)

(B) 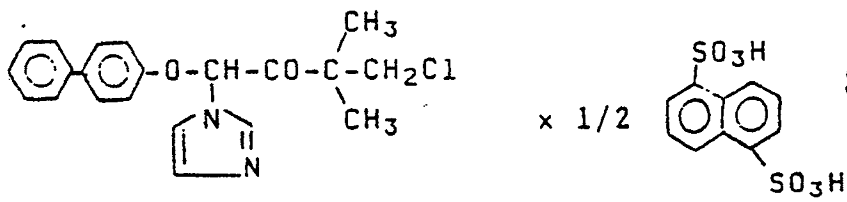

× 1/2

gemäß DE-A-2632602 (Beispiel 5)

(C) 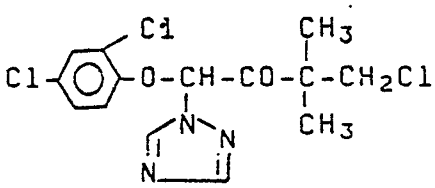 gemäß DE-A-2632603 (Beispiel 6)

(D) 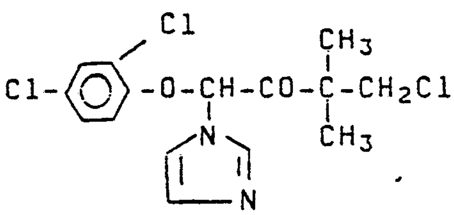 gemäß DE-A-2632602 (Beispiel 7)

**Beispiel A**

Podosphaera-Test (Apfel) / protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (podosphaera leucotricha) inokuliert.

Die Pflanzen werden dann im Gewächshaus bei 23°C und einer relativen Luftfeuchtigkeit von ca. 70 % aufgestellt.

9 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1,2,4,6,15,5 und 9.

**Beispiel B**

Sphaerotheca-Test (Gurke) / protektiv
Lösungsmittel: 4,7 Gewichtsteile Aceton
Emulgator: 0,3 Gewichtsteile Alkylerylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das-Konzentrat mit Wasser auf die gewünchte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca.75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele. 2,15,6, 5 und 9.

**Beispiel C**

Erysiphe-Test (Gerste) / protektiv
Lösungsmittel: 100 Gewichtsteile Dimethylformamid
Emulgator: 0,25 Gewichtsteile Alkylarylpolyglykolether

Zur Herstellung einer zwieckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 2,15,9,1,4,5 und 6.

**Beispiel D**

Erysiphe-Test (Gerste) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 x 12 Korn 2 cm tief in eine Standarderde. 7 Tage nach der Aussaat, wenn die jungen Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Eine deutliche Überlegenheit in der Wirksamkeit gegenüber dem Stand der Technik zeigen bei diesem Test z.B. die Verbindungen gemäß folgender Herstellungsbeispiele: 2,15,9 und 4.

**Patentansprüche**

1. 5-Aryloxy-5-azolyl-3,3-dimethyl-1-penten-4-one und -ole der allgemeinen Formel (I)

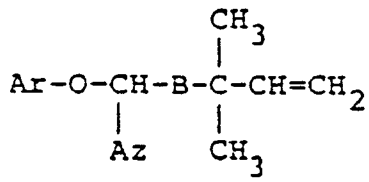

(I)

in welcher

Ar für gebenenfalls einfach oder mehrfach, gleich oder verschieden substituiertes phenyl steht, wobei als Substiuenten Halogen, Alkyl mit 1 bis 4 Kohlenstoffatomen, Halogenalkyl mit 1 bis 2 Kohlenstoff- und 1 bis 5 gleichen oder verschiedenen Halogenatomen, Alkoxycarbonyl mit 1 bis 4 Kohlenstoffatomen im Alkylteil, Nitro, Cyano oder gegebenenfalls durch Halogen und/oder Alkyl mit 1 bis 2 Kohlenstoffatomen substituiertes Phenyl genannt seien,

Az für 1,2,4-Triazol-1-yl, 1,2,4-Triazol-4-yl oder Imidazol-1-yl steht und

B für die Ketogruppe oder eine CH(OH)-Gruppierung steht,

sowie deren physiologisch verträgliche Säureadditionssalze und Metallsalz-Komplexe.

2. Verbindungen gemäß Anspruch 1, wobei

Ar für phenyl steht, welches ein - bis dreifach durch Fluor, Chlor, Brom, Methyl, Ethyl, Trifluormethyl, Methoxycarbonyl, Ethoxycarbonyl, Nitro, Cyano, phenyl und Chlorphenyl substituiert sein kann.

3. Verfahren zur Herstellung von 5-Aryloxy-5-azolyl-3,1-dimethyl-1-penten-4-onen und -olen der Formel (I)

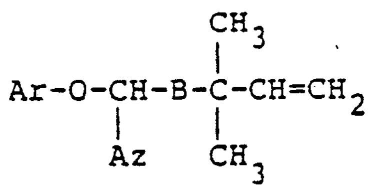

(I)

in welcher

Ar für gegebenenfalls einfach oder mehrfach gleich oder verschieden substituiertes phenyl steht, wobei als Substiuenten Halogen, Alkyl mit 1 bis 4 Kohlenstoffatomen, Halogenalkyl mit 1 bis 2 Kohlenstoff- und 1 bis 5 gleichen oder verschiedenen Halogenatomen, Alkoxycarbonyl mit 1 bis 4 Kohlenstoffatomen im Alkylteil, Nitro, Cyano oder gegebenenfalls durch Halogen und/oder Alkyl mit 1 bis 2 Kohlenstoffatomen substituiertes phenyl genannt seien,

Az für 1,2,4-Triazol-1-yl, 1,2,4-Triazol-4-yl oder Imidazol-1-yl steht und

B für die Ketogruppe oder eine CH(OH)-Gruppierung steht, dadurch gekennzeichnet, daß man Halogenetherketone der Formel (II)

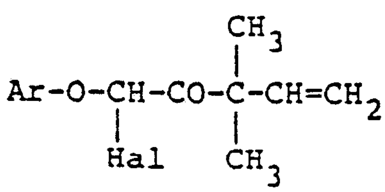

(II)

in welcher

Ar die oben angegebene Bedeutung hat und

Hal für Chlor oder Brom steht,

mit 1,2,4-Triazol oder Imidazol in Gegenwart eines Säurebindemittels und gegebenenfalls in Gegenwart eines Verdünnungsmittels umsetzt und gegebenenfalls noch die erhaltenen Keto-Derivate der Formel (I) nach bekannten Methoden in üblicher Weise reduziert, und an die so erhaltenen Verbindungen der Formel (I) gegebenenfalls noch anschließend eine Säure oder ein Metallsalz addiert.

4. Fungizide Mittel gemäß Anspruch 1, gekennzeichnet durch einen Gehalt an mindestens einem 5-Aryloxy-5-azolyl-3,3-dimethyl-1-penten-4-on oder -ol.

5. Verwendung von 5-Aryloxy-5-azolyl-3,3-dimethyl-1-penten-4-onen oder -olen gemäß Anspruch 1 zur Bekämpfung von Pilzen im Pflanzenschutz.

**Claims**

1. 5-Aryloxy-5-azolyl-3 3-dimethyl-1-penten-4-ones and -ols of the general formula (I)

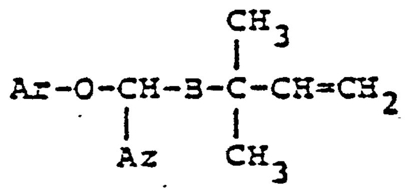

I

in which

Ar represents phenyl which is optionally mono- or polysubstituted by identical or different substituents, the following being mentioned as substituents: halogen, alkyl with 1 to 4 carbon atoms, halogenoalkyl with 1 to 2 carbon and 1 to 5 identical or different halogen atoms, alkoxycarbonyl with 1 to 4. carbon atoms in the alkyl part, nitro, cyano or phenyl which is optionally substituted by halogen and/or alkyl with 1 to 2 carbon atoms, Az represents 1,2,4-triazol-1-yl, 1,2,4-triazol-4-yl or imidazol-1-yl and

B represents the keto group or a CH(OH) grouping, and physiologically tolerated acid addition salts and metal salt complexes thereof.

2. Compounds according to Claim 1, wherein

Ar represents phenyl which can be mono- to trisubstituted by fluorine, chlorine, bromine, methyl, ethyl, trifluoromethyl, methoxycarbonyl, ethoxycarbonyl, nitro, cyano, phenyl and chlorophenyl.

3. Process for the preparation of 5-aryloxy-5-azolyl-3,3-dimethyl-1-penten-4-ones and -ols of the formula (I)

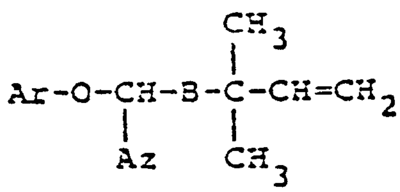

I

in which

Ar represents phenyl which is optionally mono- or polysubstituted by identical or different substituents, the following being mentioned as substituents: halogen, alkyl with 1 to 4 carbon atoms, halogenoalkyl with 1 to 2 carbon and 1 to 5 identical or different halogen atoms, alkoxycarbonyl with 1 to 4 carbon atoms in the alkyl part, nitro, cyano or phenyl which is optionally substituted by halogen and/or alkyl with 1 to 2 carbon atoms, Az represents 1,2,4-triazol-1-yl, 1,2,4-triazol-4-yl or imidazol-1-yl and

B represents the keto group or a CH(OH) grouping, characterised in that halogenoether ketones of the formula (II)

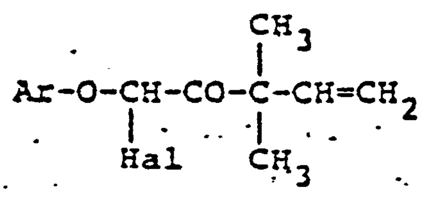

II

in which

Ar has the abovementioned meaning and

Hal represents chlorine or bromine,

are reacted with 1,2,4-triazole or imidazole in the presence of an acid-binding agent and if appropriate in the presence of a diluent and, if desired, the keto derivatives of the formula (I) obtained are also reduced in a customary manner by known methods, and, if desired, an acid or a metal salt is then also added on to the compounds of the formula (I) thus obtained.

4. Pungicidal agents according to Claim 1, characterised in that they contain at least one 5-aryloxy-5-azolyl-3,3-dimethyl-1-penten-4-one or -ol.

5. Use of 5-aryloxy-5-azolyl-3,3-dimethyl-1-penten-4-ones or -ols according to Claim 1 for combating fungi in plant protection.

**Revendications**

1. 5-aryloxy-5-azolyl-3,3-diméthyl-1-pentène-4-ones et -ols, de formule générale (I)

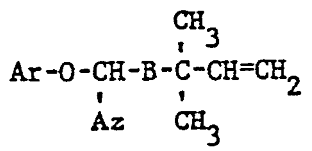

I

dans laquelle
Ar représente un groupe phényle éventuellement substitué une ou plusieurs fois, de façon identique ou différente, et l'on peut alors citer comme substituants 1 atome d'halogène, un groupe alkyle ayant 1 à 4 atomes de carbone, halogènoalkyle ayant 1 à 2 atomes de carbone et 1 à 5 atomes d'halogène(s) identiques ou différents, alcoxycarbonyle ayant 1 à 4 atomes de carbone dans la partie alkyle, nitro, cyano ou un groupe phényle éventuellement substitué par de l'halogéne et/ou un groupe alkyle ayant 1 à 2 atomes de carbone,
Az représente un groupe 1,2,4-triazol-1-yle, 1,2,4-triazol-4-yle ou imidazol-1-yle, et
B représente le groupe cétone ou un groupement CH(OH), ainsi que leurs sels d'addition d'acides ou complexes avec des sels de métaux, physiologiquement compatibles.

2. Composés selon la revendication 1, dans lesquels
Ar représente un groupe phényle qui peut être substitué une à trois fois par du fluor, du chlore, du brome, un groupe méthyle, éthyle, trifluorométhyle, méthoxycarbonyle, éthoxycarbonyle, nitro, cyano, phényle et chlorophényle.

3. Procédé de préparation de 5-aryloxy-5-azolyl-3,3-diméthyl-1-pentène-4-ones et -ols de formule (I)

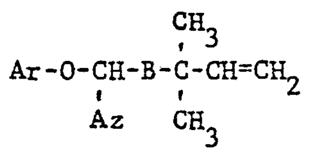

I

dans laquelle
Ar représente un groupe phényle éventuellement substitué une ou plusieurs fois, de façon identique ou différente, et l'on peut alors citer comme substituants 1 atome d'halogène, un groupe alkyle ayant 1 à 4 atomes de carbone, halogénoalkyle ayant 1 à 2 atomes de carbone et 1 à 5 atomes d'halogène(s) identiques ou diff451rents, alcoxycarbonyle ayant 1 à 4 atomes de carbone dans la partie alkyle, nitro, cyano ou un groupe phényle éventuellement substitué par de l'halogène et/ou un groupe alkyle ayant 1 à 2 atomes de carbone,
Az représente un groupe 1,2,4-triazol-1-yle, 1,2,4-triazol-4-yle ou imidazol-1-yle, et
B représente le groupe cétone ou un groupement CH(OH), caractérisé en ce qu'on fait réagir des halogéno-éther-cétones de formule (II)

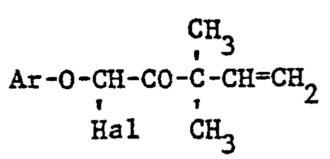

II

dans laquelle
Ar a le sens indiqué ci-dessus, et
Hal représente le chlore ou le brome,
avec le 1,2,4-triazole ou l'imidazole en présence d'un agent de fixation des acides éventuellement en présence d'un diluant, et l'on réduit éventuellement encore, de façon usuelle selon des méthodes connues, les dérivés cétoniques de formule (I) obtenus et, sur les composés ainsi obtenus de formule (I), on fixe éventuellement encore, ensuite, par addition un acide ou un sel de métal.

4. Produit fongicide selon la revendication 1, caractérisé par une teneur en au moins un 5-aryloxy-5-azolyl-3,3-diméthyl-1-pentène-4-one ou -ol.

5. Utilisation de 5-aryloxy-5-azolyl-3,3-diméthyl-1-pentène-4-ones ou -ols selon la revendication 1 pour la lutte contre les mycètes pour la protection des plantes.